# EUROPEAN PATENT APPLICATION

(11) **EP 4 509 126 A1**
(43) Date of publication of application: **19.02.2025**
(21) Application number: 23787834.3
(22) Date of filing: 14.04.2023
(51) Int. Cl.: A61K 31/58, A61K 31/575, A61P 27/10

(54) **USE OF STEROID COMPOUND IN PREPARING DRUGS FOR PREVENTING AND/OR TREATING PRESBYOPIA**

(30) Priority: 15.04.2022 CN 202210397171
(71) Applicant: Guangzhou Ocusun Ophthalmic Biotechnology Co., Ltd., Guangzhou, Guangdong 511400 (CN); Ocusun Ophthalmic Pharmaceutical (Guangzhou) Co., Ltd., Guangzhou, Guangdong 511400 (CN)
(72) Inventor: WANG, Yandong, Guangzhou, Guangdong 511400 (CN); YU, Chuiliang, Guangzhou, Guangdong 511400 (CN); SU, Yingxue, Guangzhou, Guangdong 511400 (CN); LIANG, Chiyong, Guangzhou, Guangdong 511400 (CN); CAO, Chen, Guangzhou, Guangdong 511400 (CN); LIANG, Guangjiang, Guangzhou, Guangdong 511400 (CN); YAO, Xiangchao, Guangzhou, Guangdong 511400 (CN); WU, Meirong, Guangzhou, Guangdong 511400 (CN)
(74) Representative: Calysta NV
(86) International application number: PCT/CN2023/088387
(87) International publication number: WO 2023/198192

(57) **Abstract**

The present invention provides use of a steroid compound in preparing drugs for preventing and/or treating presbyopia. The steroid compound provided by the present invention is represented by formula (I). The steroid compound has the effect of treating, alleviating and preventing presbyopia, and can greatly alleviate and/or cure presbyopia to improve vision.

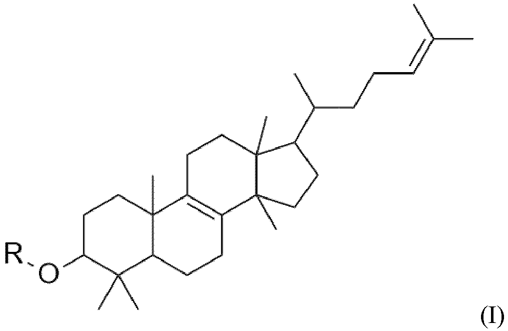

## Description

### TECHNICAL FIELD

The present invention belongs to the technical field of chemical medicine, and relates to use of a steroid compound in preparing drugs for preventing and/or treating presbyopia.

### BACKGROUND ART

Presbyopia, also known as aged eye, is a physiological condition of hardening of the lens and a decline of ocular vision accommodative function with age increasing. The initial symptom includes the need to maintain a certain distance from objects to see clearly, if the light is insufficient, reading up close will be more difficult, and these conditions will continue to worsen with age increasing. In addition to the age factor, the development of presbyopia is also related to refractive errors, physical fitness, drugs and other factors.

At present, the main treatment methods for presbyopia include wearing frame reading glasses, wearing corneal contact lenses, corneal refractive surgery, scleral refractive surgery and lens refractive surgery. In October 2021, the U.S. Food and Drug Administration (FDA) approved an ophthalmic drug Vuity (pilocarpine, 1.25% eye drops) from Allergan, a unit of AbbVie', for the treatment of presbyopia. Vuity is a topical drug that is administrated to both eyes once a day, which takes effect in 15 minutes after dripping into the eyes and has a lasting effect, and the patients can read without wearing glasses. It is the first eye drop specifically designed to treat presbyopia, but it can only alleviate symptoms of presbyopia rather than cure presbyopia completely. Therefore, there is an urgent need for other drugs that can prevent, alleviate or treat presbyopia clinically.

### SUMMARY

In view of the shortcomings of the prior art, a purpose of the present invention is to provide use of a steroid compound in the preparation of drugs for preventing and/or treating presbyopia. The steroid compound according to the present invention can have good therapeutic, alleviative, and preventive effects on presbyopia, can greatly alleviate and/or cure presbyopia, and can improve visual clarity.

To achieve this purpose, the present invention adopts the following technical solutions.

In a first aspect, the present invention provides a use of a steroid compound in the preparation of a medicament for preventing and/or treating presbyopia. The steroid compound is a compound with a structure shown in formula I, or is a stereoisomer, tautomer, nitrogen oxide, solvate, metabolite, pharmaceutically acceptable salt, or prodrug of the compound of the structure shown in formula I; 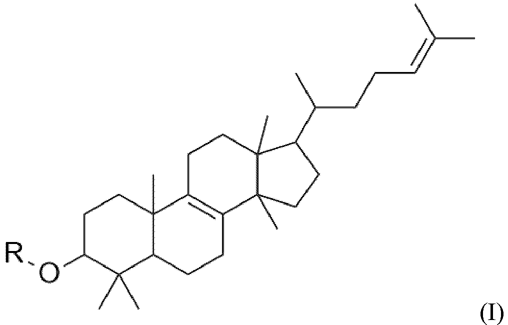
wherein, R is H, D (deuterium), alkyl, sulfate group, phosphate group, alkylsilyl, benzyl, or - C(O)-X;
X is selected from the group consisting of aryl, heteroaryl, cycloalkyl, heterocyclyl, heteroalkyl, and alkyl;
the aryl, heteroaryl, cycloalkyl, heterocyclyl, heteroalkyl, alkylsilyl and alkyl in R and X are optionally substituted by 1, 2, 3 or 4 same or different substituents;
the substituent is selected from the group consisting of D, halogen, hydroxyl, sulfhydryl, amino, cyano, nitro, carboxyl, alkylcarbonyl, alkyl, haloalkyl, cycloalkyl, heterocyclyl, aryl, arylalkyl, heteroaryl, carboxyl, R¹R²NC(=O)-, and R¹R²NC(=NH)-NR³-; and
R¹, R², and R³ are each independently selected from the group consisting of -H, -D, and alkyl.

In some embodiments, the steroid compound is a compound with a structure shown in formula II, or is a stereoisomer, tautomer, nitrogen oxide, solvate, metabolite, pharmaceutically acceptable salt, or prodrug of the compound of the structure shown in formula II; 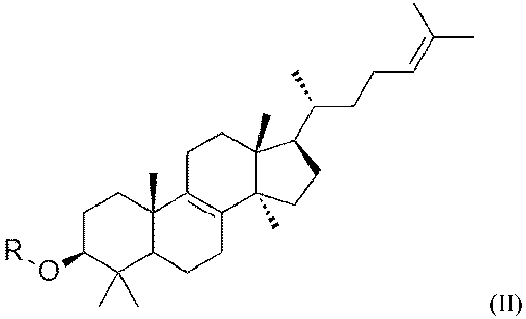 wherein, the definition of R is the same as that in formula (I).

In some embodiments, R is H, D, C₁₋₆ alkyl, sulfate group, phosphate group, C₁₋₆ alkylsilyl, benzyl, or -C(O)-X.

In some embodiments, X is selected from the group consisting of C₆₋₁₀ aryl, C₂₋₉ heteroaryl, C₃₋₈ cycloalkyl, C₂₋₁₀ heterocyclyl, C₁₋₆ heteroalkyl, and C₁₋₆ alkyl.

In some embodiments, the C₆₋₁₀ aryl, C₂₋₉ heteroaryl, C₃₋₈ cycloalkyl, C₂₋₁₀ heterocyclyl, C₁₋₆ heteroalkyl, C₁₋₆ alkylsilyl, or C₁₋₆ alkyl in R and X are optionally substituted by 1, 2, 3, or 4 same or different substituents.

In some embodiments, the substituent is selected from the group consisting of D, halogen, hydroxyl, sulfhydryl, amino, cyano, nitro, carboxyl, C₁₋₆ alkylcarbonyl, C₁₋₆ alkyl, C₁₋₆haloalkyl, C₃₋₈ cycloalkyl, C₂₋₁₀ heterocyclyl, C₆₋₁₀ aryl, C₆₋₁₀ aryl C₁₋₆ alkyl, C₂₋₉ heteroaryl, carboxyl (e.g.-COOH), R¹R²NC(=O)-, and R¹R²NC(=NH)-NR³-.

In some embodiments, R¹, R², and R³ are each independently selected from the group consisting of -H, -D, and C₁₋₆ alkyl.

In some embodiments, X is selected from the group consisting of methyl, ethyl, n-propyl, isopropyl, n-butyl, isobutyl, tert-butyl, hydroxymethyl, hydroxyethyl, mercaptomethyl, mercaptoethyl, aminomethyl, aminoethyl, aminopropyl, phenylmethyl, phenylethyl, imidazolylmethyl, carboxymethyl, carboxyethyl, methylthiomethyl, methylthioethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxy cyclopentyl, dithiocyclopentyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isoxazolyl, isoxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl, benzimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, 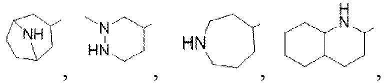 C₁₋₃ alkyl with H₂NC(=O)-substituent, and C₁₋₃ alkyl with H₂NC(=NH)-NH- substituent.

In some embodiments, the substituents in R and X are selected from the group consisting of-H, -D, halogen, hydroxyl, amino, cyano, nitro, carboxyl, C₁₋₆ alkylcarbonyl, C₁₋₆ alkyl, C₁₋₆ haloalkyl, C₃₋₈ cycloalkyl, C₂₋₉ heterocyclyl, C₆₋₁₀ aryl, and C₂₋₉ heteroaryl.

In other embodiments, the substituents in R and X are selected from the group consisting of-H, -D, -F, -Cl, -Br, hydroxyl, amino, cyano, carboxyl, formyl, acetyl, methyl, ethyl, propyl, isopropyl, n-butyl, isobutyl, tert-butyl, trifluoromethyl, difluoromethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxy cyclopentyl, dithiocyclopentyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isoxazolyl, isoxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl, benzoimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl.

As a specific embodiment of the present invention, X is selected from any one of the following compounds:

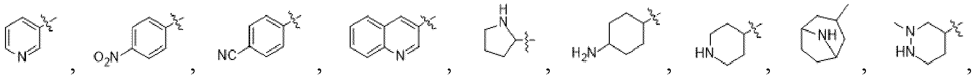

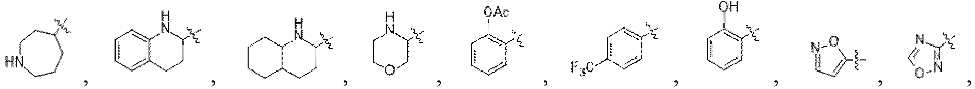

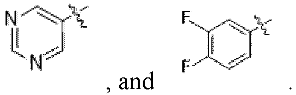

As a specific embodiment of the present invention, the compound of the structure shown in formula I or II is selected from any one of the following compounds: 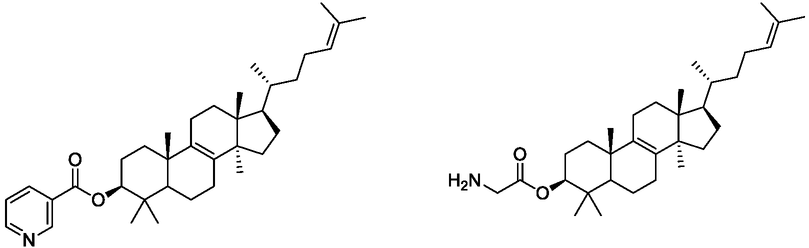 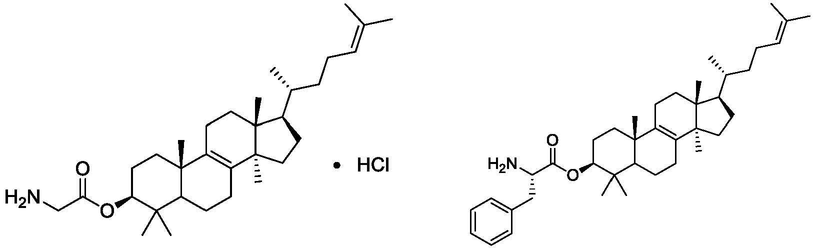 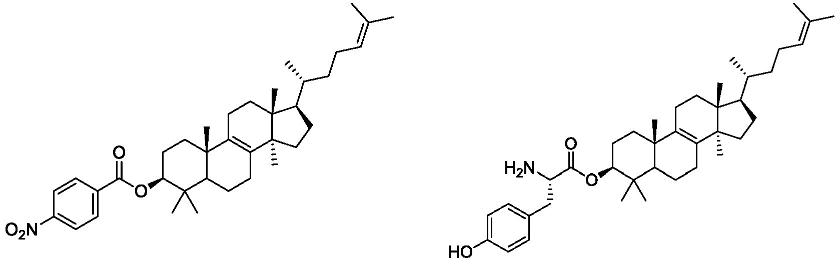 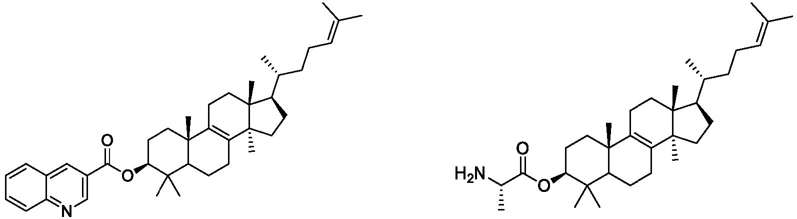 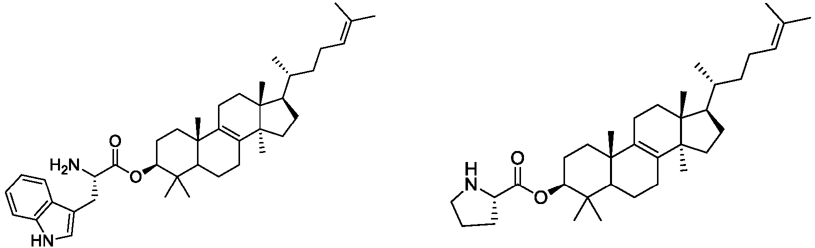 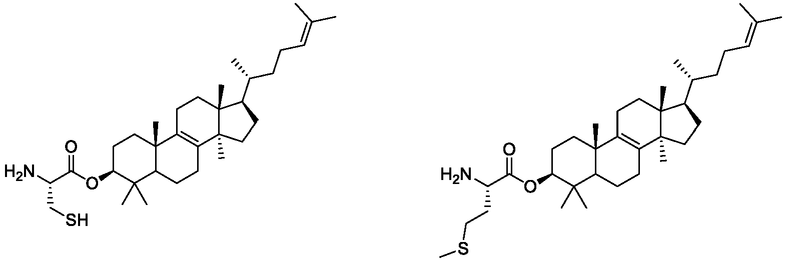 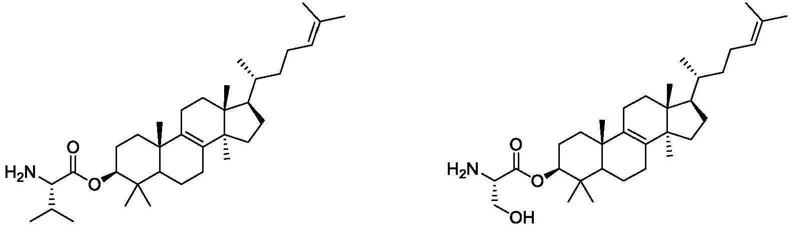 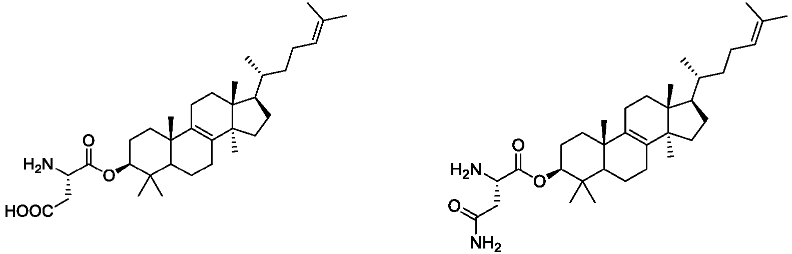 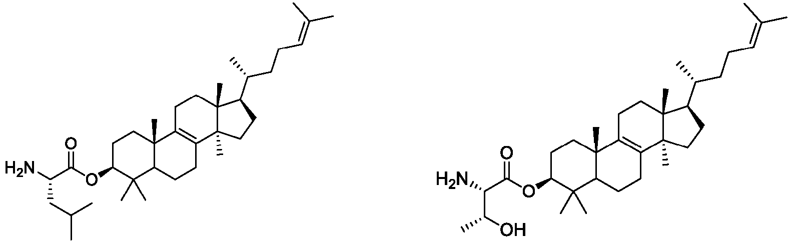 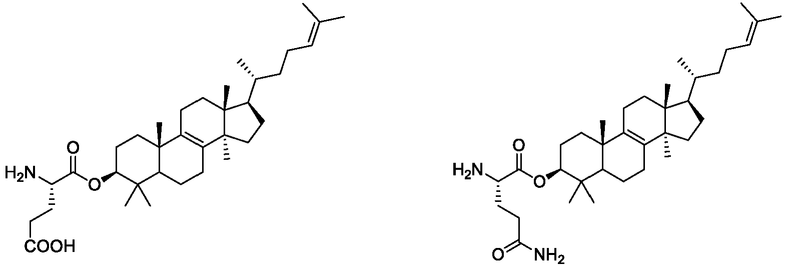 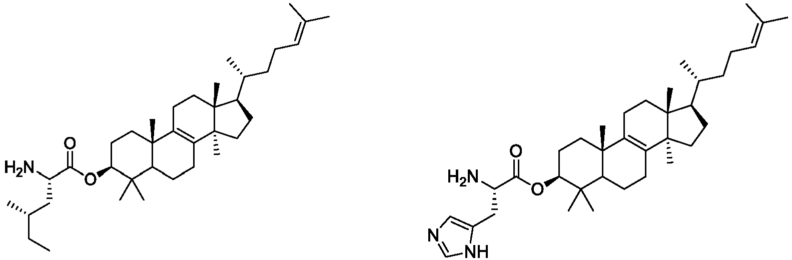 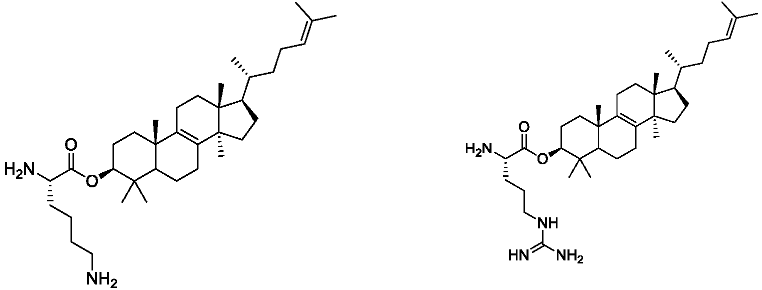 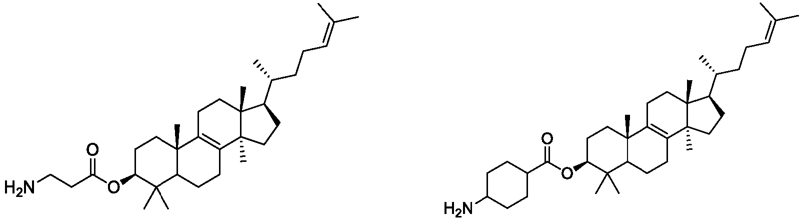 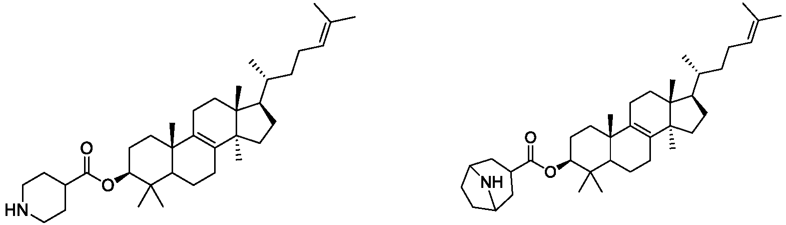 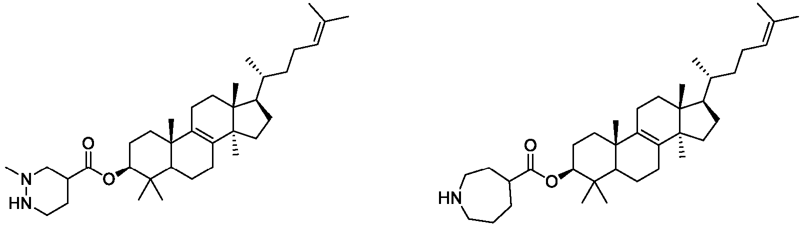 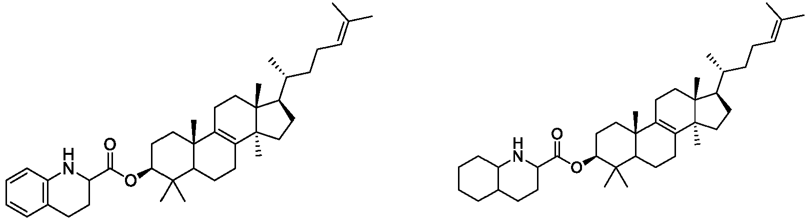 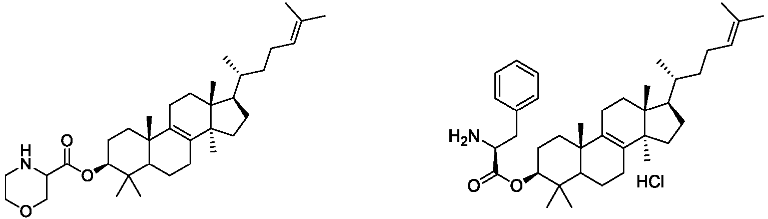 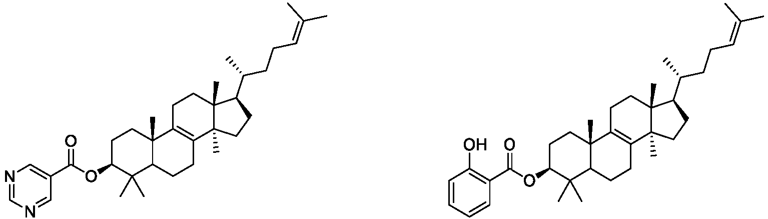 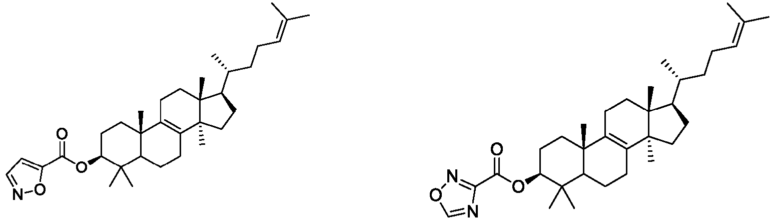 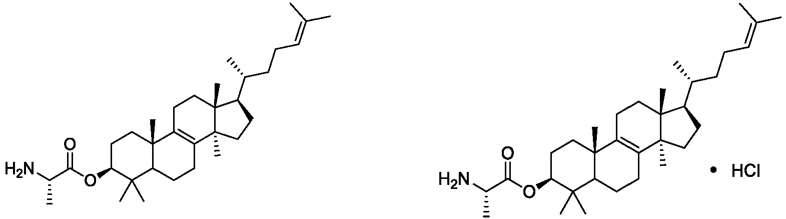 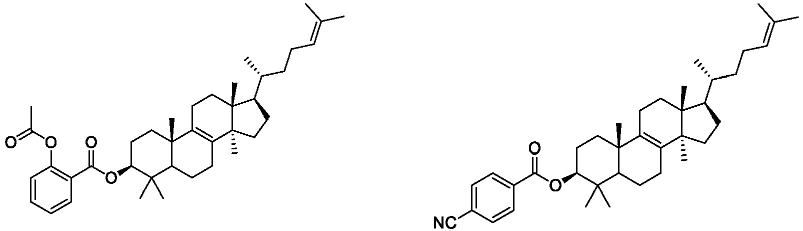 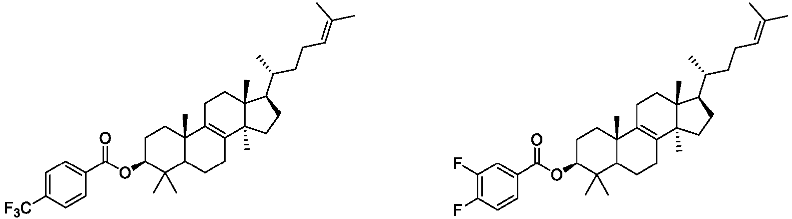 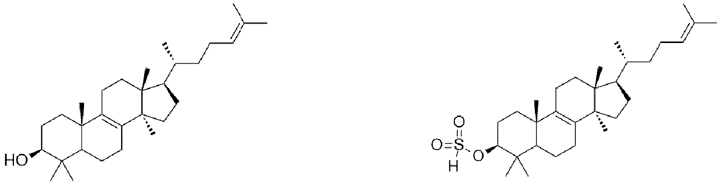 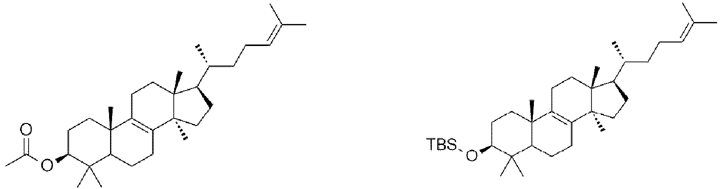 and

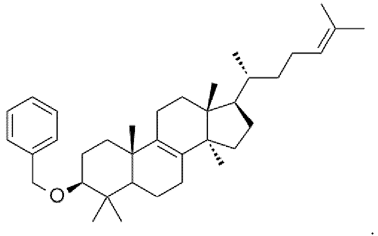

The steroid compound provided by the present invention can be used as an unprocessed chemical drug for treatment or as an active ingredient of a pharmaceutical composition.

In a second aspect, the present invention provides a use of a composition in the preparation of a medicament for preventing and/or treating presbyopia, in which the composition comprises the steroid compound of the first aspect, and one or more of pharmaceutically acceptable carriers, excipients, diluents, adjuvants, and vehicles.

Substances that can be used as pharmaceutically acceptable carriers include, but not limited to, ion exchanger; aluminum; aluminum stearate; lecithin; serum protein, such as human serum protein; buffering substance such as phosphate; glycine; sorbic acid; potassium sorbate; partial glyceride mixture of saturated vegetable fatty acid; water; salt; electrolyte, such as protamine sulfate; disodium hydrogen phosphate; potassium hydrogen phosphate; sodium chloride; zinc salt; colloidal silicon; magnesium trisilicate; polyvinylpyrrolidone; polyacrylate; wax; polyethylene-polyoxypropylene-blocking polymer; lanolin; sugar, such as lactose, glucose and sucrose; starch, such as corn starch and potato starch; cellulose and derivative thereof, such as sodium carboxymethylcellulose, ethyl cellulose and cellulose acetate; gum powder; malt; gelatin; talcum powder; excipient such as cocoa bean butter and suppository wax; oils, such as peanut oil, cottonseed oil, safflower oil, sesame oil, olive oil, corn oil and soybean oil; diol based compounds, such as propylene glycol and polyethylene glycol; esters, such as ethyl oleate and ethyl laurate; agar; buffer agent, such as magnesium hydroxide and aluminum hydroxide; alginic acid; pyrogen free water; isotonic salt; Ringer's solution; ethanol, phosphate buffered solutions, and other non-toxic suitable lubricants such as sodium laurylsulfate and magnesium stearate, colorants, releasers, coating materials, sweeteners, flavorings and fragrances, preservatives and antioxidants.

In some embodiments, the composition comprises: the steroid compound of the present invention, hydroxypropyl methylcellulose, Poloxamer 407, Poloxamer 188, and water.

In some embodiments, the composition comprises: 10 to 40 parts by mass of the steroid compound provided by the present invention, 110 to 120 parts by mass of hydroxypropyl methylcellulose, 2050 to 2060 parts by mass of Poloxamer 407, 160 to 170 parts by mass of Poloxamer 188, and 10,000 parts by mass of water.

In some embodiments, the composition can be an eye drop. The eye drop can be suspension eye drop. In some embodiments of the present invention, the specification of the eye drop can be 10 mL.

In a third aspect, the present invention provides a method for preventing and/or treating presbyopia, comprising administering the steroid compound of the first aspect or the composition of the second aspect to a subject in need thereof.

Compared with the prior art, the present invention has the following beneficial effects:
It is found in the present invention that the steroid compound provided by the present invention can improve the elasticity of the lens, improve the near vision of the patient, and can be used to prevent, treat, cure or alleviate the presbyopia in patients, and the side effects are minimal or basically free.

### DETAILED DESCRIPTION OF THE EMBODIMENT

The technical solution of the present invention is further described below by specific embodiments. Those skilled in the art should understand that the specific embodiments are merely intended to assist in understanding the present invention and should not be regarded as a specific limitation to the present invention.

Unless otherwise indicated, all scientific and technical terms used in the present invention have the same meaning as commonly understood by those skilled in the art to which the present invention pertains.

The term "stereoisomer" mentioned in the present invention refers to a compound with the same chemical structure, but the atoms or groups are arranged differently in space. Stereoisomers include enantiomers, diastereomers, conformational isomers (rotational isomers), geometric isomers (cis/trans isomers), rotational resistance isomers, and the like.

Depending on the choice of starting materials and methods, the compounds of the present invention can exist in the form of one of the possible isomers or mixtures of them, such as mixtures of racemes and diastereomers (depending on the number of asymmetric carbon atoms). Optically active (R)- or (S) -isomers can be prepared using chiral synthons or chiral reagents or resolved using conventional techniques. If the compound contains a double bond, the substituent can be in an E or Z configuration; if the compound contains a disubstituted cycloalkyl, the substituents of the cycloalkyl can be in cis- or trans-configuration.

Any resulting mixture of stereoisomers can be separated into pure or essentially pure geometric isomers, enantiomers, or diastereomers depending on differences in the physical and chemical properties of the components, for example, by chromatography and/or fractional crystallization.

Unless otherwise indicated, the structural formula described herein includes all isomer forms (such as enantiomers, diastereomers, and geometric isomers (or conformational isomers): such as R and S configurations containing asymmetric centers, (Z) and (E) isomers of double bonds, as well as (Z) and (E) conformational isomers. Therefore, a single stereochemical isomer of the compound in the present invention or the mixtures of enantiomer, diastereomer, or geometric isomers (or conformational isomers) thereof fall within the scope of the present invention.

The term "prodrug" used in the present invention represents a compound which can convert to the compound shown in formula I *in vivo.* Such conversion is influenced by the hydrolysis of the prodrug in the blood or the enzymatic conversion of the prodrug to the parent structure in the blood or tissues. The prodrug compound of the present invention can be an ester, which in the present invention can be used as a prodrug, including phenyl esters, aliphatic (C₁₋₂₄) esters, acyloxymethyl esters, carbonic esters, carbamate esters, and amino acid esters. For example, a compound of the present invention contains a hydroxyl, such compound can be acylated to form a prodrug. Other forms of prodrug include phosphate esters, such phosphate ester compounds are obtained by the phosphorylation of the parent hydroxyl group. A complete discussion of prodrug can be found in the following literatures: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al, Prodrugs: Design and Clinical Applications, Nature Review Drug Discovery, 2008, 7, 255-270, and S. J. Hecker et al, Prodrugs of Phosphates and Phosphonates, Journal of Medicinal Chemistry, 2008, 51, 2328-2345.

The racemates of any resulting final product or intermediate can be separated into optical enantiomers by a known method familiar to those skilled in the art, for example, by the separation of obtained salts of diastereomers. Racemic products can also be separated by chiral chromatography, such as high-performance liquid chromatography (HPLC) with chiral adsorbents. In particular, enantiomers can be prepared by asymmetric synthesis, for example, by reference to: Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers with different energies that can be converted to each other through a low energy barrier. If tautomerism is possible (e.g. in solution), chemical equilibrium of the tautomer can be achieved. For example, proton tautomers (also known as prototropic tautomers) include interconversion by proton transfer, such as keto-enol isomerization and imine-enamine isomerization. A valence tautomer includes interconversion through the recombination of some bond-forming electrons. A specific example of keto-enol tautomerism is the interconversion between two tautomerism of pentane-2,4-diketone and 4-hydroxypentyl-3-ene-2-ketone. Another example of tautomerism is phenol-ketone tautomerism. A specific example of phenol-ketone tautomerism is the tautomerism of pyridine-4-alcohol and pyridine-4(1H)-ketone tautomerism. Unless otherwise indicated, all tautomeric forms of the compounds of the present invention fall within the scope of the present invention.

The salts mentioned in the present invention are pharmaceutically acceptable salts, in which the term "pharmaceutically acceptable salts" is well known in the art, as recorded in the literature: Berge et al., describe pharmaceutically acceptable salts in detail in J. Pharmacol Sci, 1997, 66, 1-19. Nonlimiting examples of pharmaceutically acceptable salts include inorganic salts formed by reaction with an amino group, such as hydrochloride, hydrobromide, phosphate, metaphosphate, sulfate, sulfite, nitrate and perchlorate, and organic salts, such as carboxylate, sulfonate, sulfinate, thiocarboxylate, and the like, specifically such as, but not limited to, methanesulfonate, ethanesulfonate, formate, acetate, succinate, benzoate, succinate, dihydroxynaphthoate, salicylate, galactodicate, glucoheptanate, mandelate, 1,2-ethanedisulfonate, 2-naphthalenesulfonate, carbonate, trifluoroacetate, hydroxyacetate, hydroxyethyl sulfonate, oxalate, maleate, tartrate, citrate, succinate, malonate, benzenesulfonate, p-toluenesulfonate, malate, fumarate, lactate, lactobionate, or oxalic acid, or such salts obtained by other methods such as ion exchange method described in books and literatures. Other pharmaceutically acceptable salts include adipate, alginate, ascorbate, aspartate, benzenesulfonate, bisulfate, borate, butyrate, camphorate, camphor sulfonate, cyclopentylpropionate, disgluconate, dodecyl sulfate, ethanesulfonate, glucoheptanate, glycerol phosphate, gluconate, hemisulfate, heptanate, caproate, hydroiodate, 2-hydroxy-ethanesulfonate, lacturonate, laurate, lauryl sulfate, nicotinate, nitrate, oleate, palmitate, pamoate, pectinate, persulfate, 3-phenylpropionate, picrate, pivalate, propionate, stearate, thiocyanate, undecanoate, valerate, and the like. In addition, pharmaceutically acceptable salts also include salts obtained from appropriate bases, such as salts of alkali metals, alkaline earth metals, ammonium, and N⁺(C₁₋₄ alkyl)₄. The present invention also intends to conceive a quaternary ammonium salt formed by any compound containing a group of N. Water-soluble or oil-soluble or dispersed products can be obtained by quaternization. Alkali metals or alkaline earth metal salts include sodium, lithium, potassium, calcium, magnesium, and the like. Pharmaceutically acceptable salts further include appropriate, non-toxic ammonium, quaternary ammonium salts, and amine cations that formed by resist equilibrium ion, such as halides, carboxylates, sulfates, phosphonates, nitrates, C₁₋₈ sulfonates, and aromatic sulfonates.

The pharmaceutically acceptable salts can be formed with inorganic and organic acids, for examples such salts include acetate, aspartate, benzoate, benzenesulfonate, bromide/hydrobromide, bicarbonate/carbonate, bisulfate/sulfate, camphor sulfonate, chloride/hydrochloride, chlorophylline salts, citrate, ethanedisulfonate, fumarate, glucoheptanate, gluconate, glucuronate, hippurate, hydroiodate/iodide, hydroxyethyl sulfonate, lactate, lacturonate, lauryl sulfate, malate, maleate, malonate, mandelate, methanesulfonate, methyl sulfate, naphthalate, naphthalene sulfonate, nicotinate, nitrate, octadecate, oleate, oxalate, palmitate, pamoate, phosphate/hydrogen phosphate/dihydrogen phosphate, polygalactodate, propionate, stearate salt, succinate, sulfosalicylate, tartrate, toluenesulfonate, and trifluoroacetate.

Inorganic acids from which salts can be derived include, for example, hydrochloric acid, hydrobromic acid, sulfuric acid, nitric acid, phosphoric acid, and the like.

Organic acids from which salts can be derived include such as acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, p-toluenesulfonic acid, sulfosalicylic acid, and the like.

The term "solvate" of the present invention refers to an associate formed by one or more solvent molecules with the compound of the present invention. Solvents that form solvates include, but not limited to, water, isopropanol, ethanol, methanol, dimethyl sulfoxide, ethyl acetate, acetic acid, and aminoethanol. The term "hydrate" refers to an associate formed by water as a solvent molecule.

The term "pharmaceutical composition" means a mixture of one or more of the compounds, salts or physiologically/pharmaceutically acceptable salts or prodrugs thereof described herein, and other chemical components, such as physiologically/pharmaceutically acceptable carriers or excipients. The purpose of a pharmaceutical composition is to facilitate the administration of a compound to an organism.

As used in the present invention, the term "treatment" of any disease or condition, in some embodiments of the present invention refers to the improvement of a disease or condition (i.e., alleviating or preventing or relieving the progression of a disease or at least one of clinical symptoms thereof). In other embodiments, "treatment" refers to the moderation or improvement of at least one physical parameter, including one that may not be perceived by the patient. In other embodiments, "treatment" refers to the regulation of a disease or condition either physically (e.g. stabilizing a perceptible symptom) or physiologically (e.g. stabilizing a physical parameter) or both. In other embodiments, "treatment" refers to the prevention or delay of the onset, occurrence, or exacerbation of a disease or condition.

The term "alkyl" used in the present invention refers to a monovalent hydrocarbon group having 1-20 carbon atoms, or 1-10 carbon atoms, or 1-8 carbon atoms, or 1-6 carbon atoms, or 1-4 carbon atoms, or 1-3 carbon atoms in a saturated linear chain or branched chain, in which the alkyl group can be independently and optionally substituted by one or more substituents described in the present invention. Examples of alkyl groups include, but not limited to, methyl (Me, -CH₃), ethyl (Et,-CH₂CH₃), n-propyl (n-Pr, -CH₂CH₂CH₃), isopropyl (i-Pr, -CH(CH₃)₂), n-butyl (n-Bu,-CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, and the like. The term "alkyl group" and its prefix "alkyl" are used herein, and both include linear and branched saturated carbon chains. The term "alkylene" used herein refers to a saturated divalent hydrocarbon group obtained by the elimination of two hydrogen atoms from linear or branched saturated hydrocarbons, such examples include, but not limited to, methylene, ethylene, isopropylene, and the like.

The term "cycloalkyl" refers to a monovalent or polyvalent, non-aromatic, saturated or partially unsaturated ring that does not contain heteroatoms, including a monocyclic with 3-12 carbon atoms or a bicyclic ring with 7-12 carbon atoms. A bicyclic carbon ring with 7-12 atoms can be a bicyclic [4,5], [5,5], [5,6] or [6,6] system, and a bicyclic carbon ring with 9 or 10 atoms can be a bicyclic [5,6] or [6,6] system. Suitable cyclic aliphatic groups include, but not limited to, cycloalkyl, cycloalkenyl, and cycloalkynyl. Examples of cyclic aliphatic groups include, but not limited to, cyclopropyl, cyclobutyl, cyclopentyl, 1-cyclopentyl-1-alkenyl, 1-cyclopentyl-2-alkenyl, 1-cyclopentyl-3-alkenyl, cyclohexyl, 1-cyclohexyl-1-alkenyl, 1-cyclohexyl-2-alkenyl, 1-cyclohexyl-3-alkenyl, cyclohexadienyl, cycloheptyl, cyclooctyl, cyclononyl, cyclodecyl, cycloundecyl, cyclododecyl, and the like. Additionally, the "cyclic aliphatic group" or "carbon ring", "carbon ring group" or "cycloalkyl" can be substituted or unsubstituted, in which the substituents can be, but not limited to, hydroxyl, amino, halogen, cyano, aryl, heteroaryl, alkoxy, alkylamino, alkyl, alkenyl, alkynyl, cycloalkyl, heterocyclyl, sulfhydryl, nitro, aryloxy, hydroxyl- substituted alkoxy, hydroxyl-substituted alkyl-C(=O), alkyl-C(=O), alkyl-S(=O), alkyl-S(=O)₂-, hydroxyl- substituted alkylS(=O), hydroxyl- substituted alkyl-S(=O)₂, carboxylalkoxy, and the like.

The terms "heterocycle", "heterocyclyl", "heteralicyclic group" or "heterocyclic" are used interchangeably herein, all referring to a monocyclic, bicyclic, and tricyclic system, in which one or more carbon atoms in the ring are independently and optionally replaced by a heteroatom, the heteroatom has the meanings defined in the present invention, and the ring can be fully saturated or contain one or more units of unsaturation, while it's not an aromatic family, and there's only one attachment site to the rest of a molecule. The hydrogen atoms on one or more rings are independently and optionally replaced by one or more substituents described in the present invention. Some of these embodiments are that the "heterocycle", "heterocyclyl", "heteralicyclic group" or "heterocyclic" group is a monocyclic ring of a 3-7 membered ring (1-6 carbon atoms and 1-3 heteroatoms selected from N, O, P and S, in which S or P is optionally replaced by one or more oxygen atoms to give groups such as SO, SO₂, PO, POz; when the ring is a tricyclic ring, in which there is only one heteroatom), or a 7-10-membered bicyclic ring (4-9 carbon atoms and 1-3 heteroatoms selected from N, O, P and S, in which S or P is optionally replaced by one or more oxygen atoms to give groups such as SO, SO₂, PO, POz).

Heterocyclyl can be carbon group or heteroatomic group. "Heterocyclyl" also includes the group formed by the combination of a heterocyclic group with a saturated or partially unsaturated ring or heterocyclic ring. Examples of heterocyclic ring include, but not limited to, pyrrolidyl, tetrahydrofuranyl, dihydrofuranyl, tetrahydrothiophenyl, tetrahydropyranyl, dihydropyranyl, tetrahydrothiopyranyl, piperidinyl, morpholinyl, thiomorpholinyl, thioxanyl, thiazolidinyl, oxazolidinyl, piperazinyl, homopiperazinyl, azacyclobutyl, oxacyclobutyl, thiacyclobutyl, piperidyl, homopiperidyl, epopropyl, azacycloheptyl, oxacycloheptyl, thiacycloheptyl, 4-methoxy-piperidin-1-yl, 1,2,3,6-tetrahydropyridin-1-yl, oxazepinyl, diazepinyl, thiazepinyl, pyrrolino-1-yl, 2-pyrrolinyl, 3-pyrrolinyl, dihydroindolyl, 2H-pyranyl, 4H-pyranyl, dioxacyclohexyl, 1,3-dioxy-amyl, pyrazolinyl, dithianyl, dithiolanyl, dihydrothiophenyl, pyrazolidinyl imidazolinyl, imidazolidinyl, 1,2,3,4-tetrahydroisoquinolinyl, 1,2,6-thiadiazinanyl 1,1-dioxy-2-yl, 4-hydroxy-1,4-azaphosphorane-4-oxide-1-yl, 2-hydroxy-1-(piperazine-1-yl) ethylketon-4-yl, 2-hydroxy-1-(5,6-dihydro-1,2,4-triazin-1(4H)-yl) ethylketon-4-yl, 5,6-dihydro-4H-1,2,4-oxadiazin-4-yl, 2-hydroxy-1-(5,6-dihydropyridin-1(2H)-yl) ethylketon-4-yl, 3-azabicyclo[3.1.0]hexyl, 3-azabicyclo[4.1.0]heptyl, azabicyclo[2.2.2]hexyl, 2-methyl-5,6,7,8-tetrahydro-[1,2,4]triazole[1,5-c]pyrimidin-6-yl, 4,5,6,7-tetrahydroisoxazol[4,3-c]pyridin-5-yl, 3H-indolyl 2-oxy-5-azabicyclo[2.2.1]heptan-5-yl, 2-oxy-5-azabicyclo[2.2.2]octan-5-yl, quinazinyl and N-pyridyl urea. Examples of heterocyclic group further include 1,1-dioxo-thiomorpholinyl, in which the two carbon atoms in the ring are replaced by oxygen atoms such as pyrimidine diketol. Additionally, the heterocyclic group can be substituted or unsubstituted, in which the substituents can be, but are not limited to, oxo (=O), hydroxyl, amino, halogen, cyano, heteroaryl, alkoxy, alkylamino, alkyl, alkenyl, alkynyl, heterocyclyl, sulfhydryl, nitro, aryloxy, hydroxyl substituted alkoxy, hydroxyl substituted alkyl-C(=O), alkyl-C(=O), alkyl-S(=O), alkyl-S(=O)z-, hydroxyl substituted alkylS(=O), hydroxyl substituted alkyl-S(=O)z, carboxyl alkoxy, and the like.

The term "aryl" can be used alone, or as a large moiety of "arylalkyl", "arylalkoxy" or "aryloxyalkyl" refers to a monocyclic, bicyclic, and tricyclic carbon ring system containing a total of 6 to 14 membered rings, in which at least one of the ring systems is aromatic, and in which each ring system contains 3 to 7 membered rings with only one attachment site connected to the rest of the molecules. The term "aryl" can be used interchangeably with the term "aromatic ring"; for example, aromatic rings can include phenyl, naphthyl, and anthryl. Additionally, the aryl can be substituted or unsubstituted, in which the substituents can be, but not limited to, hydroxyl, amino, halogen, cyano, aryl, heteroaryl, alkoxy, alkylamino, alkyl, alkenyl, alkynyl, heterocyclyl, sulfhydryl, nitro, aryloxy, hydroxyl substituted alkoxy, hydroxyl substituted alkyl-C(=O), alkyl-C(=O), alkyl-S(=O), alkyl-S(=O)z-, hydroxyl substituted alkyl-S(=O), hydroxyl substituted alkylS(=O)z, carboxyl alkoxy, and the like.

The term "heteroaryl" refers to a monocyclic, bicyclic, and tricyclic system containing a total of 5 to 14 membered rings, in which at least one ring system is aromatic, and at least one ring system contains one or more heteroatoms, in which the heteroatom has the meanings defined in the present invention, and in which each ring system contains 3 to 7 membered rings with only one attachment site connected to the rest of the molecules. The term "heteroaryl" can be used interchangeably with the term "heteroaromatic ring" or "heteroaromatics". Additionally, the heteroaryl can be substituted or unsubstituted, in which the substituents can be, but not limited to, hydroxyl, amino, halogen, cyano, aryl, heteroaryl, alkoxy, alkylamino, alkyl, alkenyl, alkynyl, cycloalkyl, heterocyclyl, sulfhydryl, nitro, aryloxy, hydroxyl substituted alkoxy, hydroxyl substituted alkyl-C(=O)-, alkyl-C(=O)-, alkyl-S(=O)-, alkyl-S(=O)₂-, hydroxyl substituted alkyl-S(=O)-, hydroxly substituted alkylS(=O)₂-, carboxyl alkoxy, and the like.

In other embodiments, heteroaryl includes, but not limited to, the following monocyclic rings: 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, 4-methylisoxazol-5-yl, N-pyrrolyl, 2-pyrrolyl, 3-pyrrolyl, 2-pyridinyl, 3-pyridinyl, 4-pyridinyl, 2-pyrimidinyl, 4-pyrimidinyl, pyrimidin-5-yl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thienyl, pyrazolyl (e.g., 2-pyrazolyl), isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, 1,3,4-thiadiazolyl-2-yl, pyrazinyl, pyrazin-2-yl, 1,3,5-triazinyl; also includes, but by no means limited to, the following bicyclic rings: benzimidazolyl, benzofuranyl, benzothiophenyl, indolyl (e.g., 2-indolyl), purinyl, quinolinyl (e.g., 2-quinolinyl, 3-quinolinyl, 4-quinolinyl), isoquinolinyl (e.g., 1-isoquinolinyl, 3-isoquinolinyl, or 4-isoquinolinyl), benzo[d]thiazol-2-yl, and imidazolazo[1,5-α]pyridin-6-yl.

The term "heteroatom" refers to one or more of O, S, N, P, and Si atoms, including N, S, and P in any oxidation state form; forms of primary, secondary, tertiary amines and quaternary ammonium salts; or the form in which the hydrogen on the nitrogen atom in the heterocyclic ring is substituted, for example, N (e.g., N in 3,4-dihydro-2H-pyrrolyl), NH (e.g., NH in pyrrolidinyl) or NR (e.g., NR in N-substituted pyrrolidinyl).

The term "heteroalkyl" refers to that one or more heteroatoms can be inserted in the middle of an alkyl chain, in which the alkyl and heteroatom have the meanings defined in the present invention. Unless otherwise specified, a heteroalkyl group contains 1-10 carbon atoms, other embodiments are that a heteroalkyl group contains 1-8 carbon atoms, other embodiments are that a heteroalkyl group contains 1-6 carbon atoms, other embodiments are that a heteroalkyl group contains 1-4 carbon atoms, and other embodiments are that a heteroalkyl group contains 1-3 carbon atoms. Such examples include, but not limited to, CH₃OCH₂-, CH₃CH₂OCH₂-, CH₃SCH₂-, CH₃SCH₂CH₂-, (CH₃)₂NCH₂-, (CH₃)₂CH₂OCH₂-, CH₃OCH₂CH₂-, CH₃CH₂OCH₂CH₂-, and the like.

The term "halogen" refers to F, Cl, Br or I.

The term "halo" in the present invention means substituting a subsequent group with halogen, and the number of substitutions can be one or more.

The term "hydroxyl substituted" in the present invention means substituting a subsequent group with hydroxy, and the number of substitutions can be one or more.

When the term "substituted" of the present invention is used between two groups, the precedent one is a substituent. For example, "aryl substituted alkyl" means that the alkyl has an aryl substituent, and "alkoxy carbonyl substituted alkyl " means that the alkyl has an alkoxycarbonyl substituent.

When multiple groups of the present invention are used in combination, from left to right, the substitution relationship is sequential, such as "arylalkyl", indicating an aryl substituted alkyl, and "alkoxyalkoxy", indicating an alkoxy substituted alkoxy.

### Example 1

The present invention provides an eye drop, which was composed of the following components shown in Table 1.

**Table 1**

| Component | Prescription 1 | Prescription 2 |
|---|---|---|
| 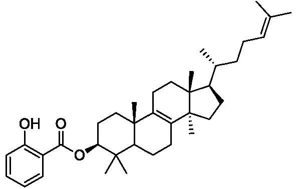 | 20 mg | 40 mg |
| HPMC E5 | 0.12 g | 0.12 g |
| Poloxamer P407 | 2.05 g | 2.05 g |
| Poloxamer P188 | 0.16 g | 0.16 g |
| water | Added to 10 mL | Added to 10 mL |

The preparation method was as follows:
Under aseptic operation, the above components were mixed and stirred at 2 to 8°C overnight, to obtain a homogenized mixture, and then stored in an eye medicine bottle.

### Example 2. Test method for presbyopia effect

Patients with presbyopia were recruited and, after signing informed consent, were administered with the eye drops prepared according to Example 1, one drop per eye at a time, four times a day, for 8 weeks. Vision tests were performed prior to administration (V1, baseline) and at 2 (V2), 4 (V3), and 8 (V4) weeks after administration to monitor the improvement/treatment of patients' presbyopia.

An ETDR near vision chart was used to check the patient's improvement in naked eye near vision (UCNVA).

The test results are listed in Table 2 below.

**Table 2**

| Evaluated eye | Gender /age | V1 (1 week) | V2 (2 weeks) | V3 (4 weeks) | V4 (8 weeks) | V2-V1 | V3-V1 | V4-V1 |
|---|---|---|---|---|---|---|---|---|
| P05 (S006) right eye | Female /65 | 29 | 33 | 35 | 40 | 4 | **6** | **11** |
| P13 (S018) right eye | Male /66 | 30 | 41 | 41 | 41 | **11** | **11** | **11** |
| P22 (S029) right eye | Female /70 | 33 | 40 | 43 | 44 | **7** | **10** | **11** |
| P25 (S034) left eye | Female /54 | 37 | 45 | 46 | 47 | **8** | **9** | **10** |
| P28 (S037) right eye | Male /54 | 50 | 61 | 60 | 65 | **11** | **10** | **15** |
| P28 (S037) left eye | Male /54 | 46 | 61 | 61 | 67 | **15** | **15** | **21** |
| P32 (S042) left eye | Female /66 | 23 | 28 | 29 | 33 | **5** | **6** | **10** |
| P36 (S045) right eye | Female /69 | 24 | 34 | 35 | 38 | **10** | **11** | **14** |
| P36 (S045) left eye | Female /69 | 26 | 33 | 35 | 39 | **7** | **9** | **13** |
| P38 (S047) left eye | Female /71 | 35 | 40 | 41 | 46 | **5** | **6** | **11** |
| P42 (S059) right eye | Female /67 | 30 | 37 | 38 | 43 | **7** | **8** | **13** |
| P42 (S059) left eye | Female /67 | 31 | 39 | 42 | 50 | **8** | **11** | **19** |
| Average | | 33 | 41 | 42 | 46 | **8** | **9** | **13** |

As can be seen from the results of Table 2, patients with different degrees of presbyopia had a great improvement in presbyopia symptoms after continuous use of the eye drops for 8 weeks, and the naked eye near vision improved by an average of about 13 letters. These results show that the eye drops of the present invention have good therapeutic effect on presbyopia patients and are safe and convenient.

### Example 3. Experiment of mouse lens elasticity test

### 1. Animals and instruments

### 1) Experimental animals

C57BL/6J male mice were purchased from Zhejiang Weitong Lihua Laboratory Animal Technology Co., LTD. Among these mice, 40 mice were 8 months old and 10 mice were 8 weeks old.

### 2) Experimental apparatus and instruments

**Table 3. Main instruments of the experiment**

| **Name** | **Supplier/Manufacturer** | **Model** |
|---|---|---|
| Weighing balance | Changzhou Keyuan Electronic Instrument Co., LTD | JY20002 |
| Electronic balance Infrared Photorefractor for small animals | METTLER TOLEDO Strai.tech | MS205DU photorefractor |
| Digital stereo microscope Pipettor | Motic Eppendorf | SMA171TLED/Moticam 2306 10 µl-K10804H |

### 2. Experimental method process

1) Administration: The administration was performed by dripping in eye with a pipettor, with 5 µL of a dripping volume in eye, only dripping in the left eye, and the frequency of administration was once a day (QD) or three times a day (TID) according to the groups. The elasticity of the mouse lens was tested after 28 days of administration.

### 2) Lens elasticity test

Lens sampling: The mice were sacrificed by excessive carbon dioxide. The eyeballs on the dripping side were extracted using ophthalmic forceps and quickly placed in a HBSS solution with 95% O₂ and 5% CO₂. After three times of rinsing, the optic nerve, sclera, cornea and retina were removed successively under asana mirror using venus scissors and microscopic forceps, and the lens were obtained and immersed in HBSS.

Preparation for shooting: The slide and cover slide (weighed, both about 0.24g) were prepared, the slide was placed under the asana mirror, and a fixed position was selected to place the lens and the subsequent cover slide.

Image acquisition: The lens was sucked out of the HBSS solution with a disposable straw and placed on a slide. After the excess liquid was quickly absorbed, the lens image (uncompressed lens image 1) was obtained with the camera attached to the asana mirror. The slide was quickly placed on the lens from a fixed position and the lens image was obtained again (the image after letting the lens stand for 2 minutes was used as the lens image 2).

Data analysis: Excel software was used to collect data. Prism (Graph pad software, Inc.) software was used for data analysis. One-way ANOVA plus Sidak comparison test data were used for comparison of lens elasticity among all groups, and p<0.05 was considered as significant difference. Lens elasticity value = lens circumference of image 2 - lens circumference of image 1.

### 3. Experimental results

In this experiment, the efficacy of prescription 2 in Example 1 in a model of spontaneous lens elasticity reduction in elderly C57BL/6 mice was evaluated.

The statistics of lens elasticity values after 28 days of administration were shown in Table 4. From the statistical values, the lens elasticity of 8-month-old mice was significantly lower than that of 8-week-old mice. In addition, compared with the vehicle control group, prescription 2 administrated once a day or 3 times a day showed a great improvement in lens elasticity.

**Table 4: Changes in lens elasticity values after four weeks of administration**

| Group | Animal condition | Concentration and frequency of administration | Elasticity of the lens mm, mean±SD | Lens elasticity improvement (compared with group 3, µm) |
|---|---|---|---|---|
| 1 | 8-week-old / 10 male | None | 0.372 ± 0.138 | / |
| 2 | 8-month-old / 10 male | None | 0.183 ± 0.178 | / |
| 3 | 8-month-old / 10 male | Vehicle, TID | 0.172 ± 0.166 | / |
| 4 | 8-month-old / 10 male | Prescription 2, QD | 0.194 ± 0.102 | 22 |
| 5 | 8-month-old / 10 male | Prescription 2, TID | 0.215 ± 0.104 | 43 |

### 4. Conclusion

In general, the lens elasticity of 8-month-old mice was significantly weaker than that of 8-week-old mice, and could be used as a model of lens elasticity reduction. The prescription 2 eye drops administrated once a day and three times a day can significantly improve the lens elasticity of 8-month-old mice, so it can be used for the prevention or treatment of presbyopia and eye diseases caused by reduced lens elasticity.

Although the present invention is described in detail by general instructions, specific embodiments and tests above, it is obvious to those skilled in the art that some modifications or improvements can be made on the basis of the present invention. Therefore, these modifications or improvements made on the basis of not deviating from the spirit of the present invention are within the scope of protection claimed by the present invention.

## Claims

1. Use of a steroid compound in the preparation of a medicament for preventing and/or treating presbyopia, wherein, the steroid compound is a compound with a structure shown in formula (I), or is a stereoisomer, tautomer, nitrogen oxide, solvate, metabolite, pharmaceutically acceptable salt or prodrug of the compound of the structure shown in formula (I); 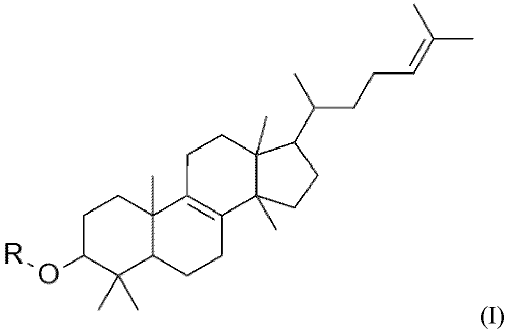
wherein, R is H, D, alkyl, sulfate group, phosphate group, alkylsilyl, benzyl, or -C(O)-X;
X is selected from the group consisting of aryl, heteroaryl, cycloalkyl, heterocyclyl, heteroalkyl, and alkyl;
the aryl, heteroaryl, cycloalkyl, heterocyclyl, heteroalkyl, alkylsilyl and alkyl in R and X are optionally substituted by 1, 2, 3 or 4 same or different substituents;
the substituent is selected from the group consisting of D, halogen, hydroxyl, sulfhydryl, amino, cyano, nitro, carboxyl, alkylcarbonyl, alkyl, haloalkyl, cycloalkyl, heterocyclyl, aryl, arylalkyl, heteroaryl, carboxyl, R¹R²NC(=O)-, and R¹R²NC(=NH)-NR³-; and
R¹, R², and R³ are each independently selected from the group consisting of H, D, and alkyl.

2. The use according to claim 1, wherein, the steroid compound is a compound with a structure shown in formula (II), or is a stereoisomer, tautomer, nitrogen oxide, solvate, metabolite, pharmaceutically acceptable salt or prodrug of the compound of the structure shown in formula (II), 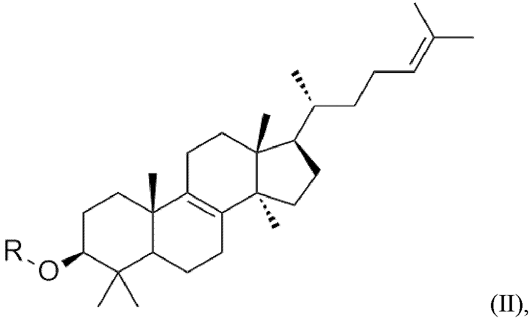 wherein, the definition of R is the same as that in formula (I).

3. The use according to claim 1, wherein, R is H, D, C₁₋₆ alkyl, sulfate group, phosphate group, C₁₋₆ alkylsilyl, benzyl, or -C(O)-X;
X is selected from the group consisting of C₆₋₁₀ aryl, C₂₋₉ heteroaryl, C₃₋₈ cycloalkyl, C₂₋₁₀ heterocyclyl, C₁₋₆ heteroalkyl, and C₁₋₆ alkyl;
the C₆₋₁₀ aryl, C₂₋₉ heteroaryl, C₃₋₈ cycloalkyl, C₂₋₁₀ heterocyclyl, C₁₋₆ heteroalkyl, C₁₋₆ alkylsilyl, or C₁₋₆ alkyl in R and X are optionally substituted by 1, 2, 3, or 4 same or different substituents;
the substituent is selected from the group consisting of D, halogen, hydroxyl, sulfhydryl, amino, cyano, nitro, carboxyl, C₁₋₆ alkylcarbonyl, C₁₋₆ alkyl, C₁₋₆ haloalkyl, C₃₋₈ cycloalkyl, C₂₋₁₀ heterocyclyl, C₆₋₁₀ aryl, C₆₋₁₀ aryl C₁₋₆ alkyl, C₂₋₉ heteroaryl, carboxyl, R¹R²NC(=O)-, and R¹R²NC(=NH)-NR³-; and
R¹, R², and R³ are each independently selected from the group consisting of H, D, and C₁₋₆ alkyl.

4. The use according to any one of claims 1 to 3, wherein, X is selected from the group consisting of methyl, ethyl, n-propyl, isopropyl, n-butyl, isobutyl, tert-butyl, hydroxymethyl, hydroxyethyl, mercaptomethyl, mercaptoethyl, aminomethyl, aminoethyl, aminopropyl, phenylmethyl, phenylethyl, imidazolylmethyl, carboxymethyl, carboxyethyl, methylthiomethyl, methylthioethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isoxazolyl, isoxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl, benzimidazolyl, benzofuranyl, benzothiophenyl, indolyl, purinyl, quinolinyl, isoquinolinyl, imidazolo[1,2-a]pyridyl, pyrazolo[1,5-a]pyridyl, pyrazolo[1,5-a]pyrimidyl, imidazolo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, 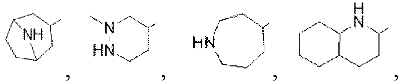 C₁₋₃ alkyl with H₂NC(=O)- substituent, and C₁₋₃ alkyl with H₂NC(=NH)-NH- substituent.

5. The use according to any one of claims 1 to 4, wherein, the substituent is selected from the group consisting of -D, halogen, hydroxyl, amino, cyano, nitro, carboxyl, C₁₋₆ alkylcarbonyl, C₁₋₆ alkyl, C₁₋₆ haloalkyl, C₃₋₈ cycloalkyl, C₂₋₉ heterocyclyl, C₆₋₁₀ aryl, and C₂₋₉ heteroaryl.

6. The use according to any one of claims 1 to 5, wherein, the substituent is selected from the group consisting of -D, -F, -Cl, -Br, hydroxyl, amino, cyano, carboxyl, formyl, acetyl, methyl, ethyl, propyl, isopropyl, n-butyl, isobutyl, tert-butyl, trifluoromethyl, difluoromethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isoxazolyl, isoxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl, benzoimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl.

7. The use according to any one of claims 1 to 6, wherein, X is selected from any one of the following compounds:

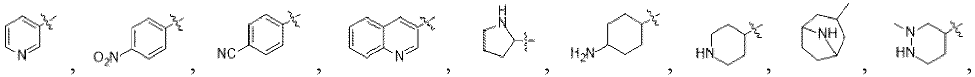

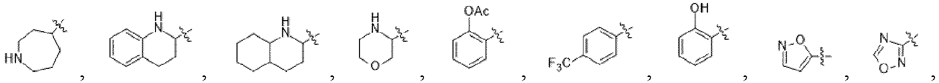

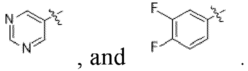

8. The use according to claim 1, wherein, the compound of the structure shown in formula (I) is selected from any one of the following compounds: 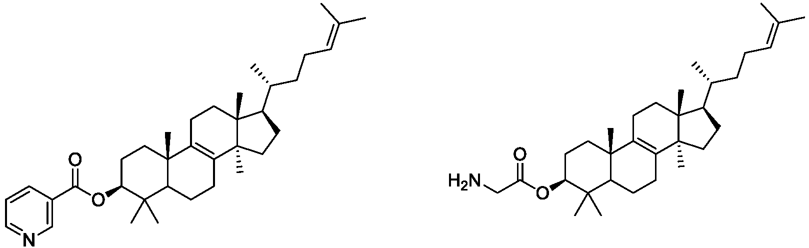 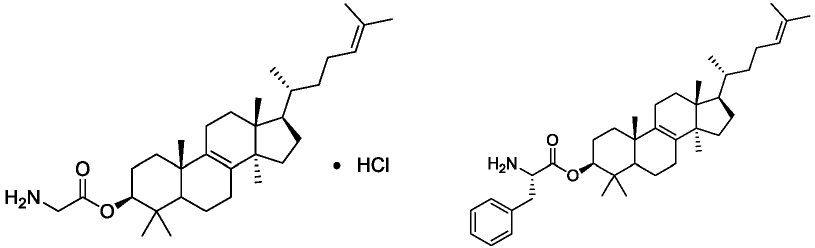 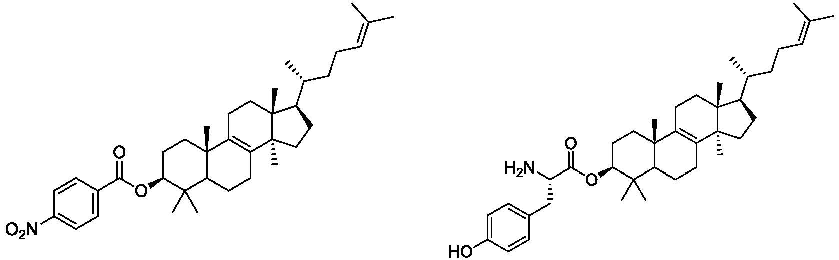 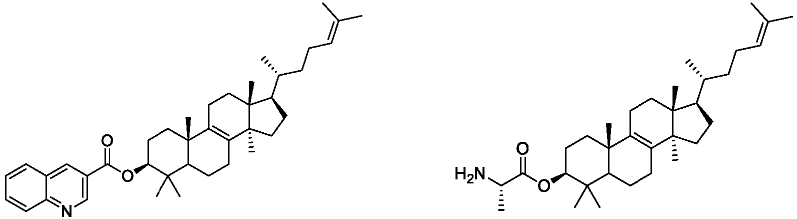 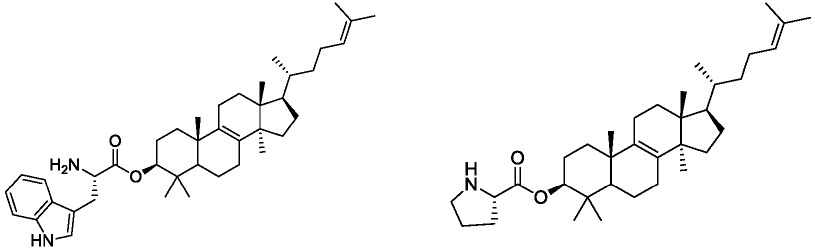 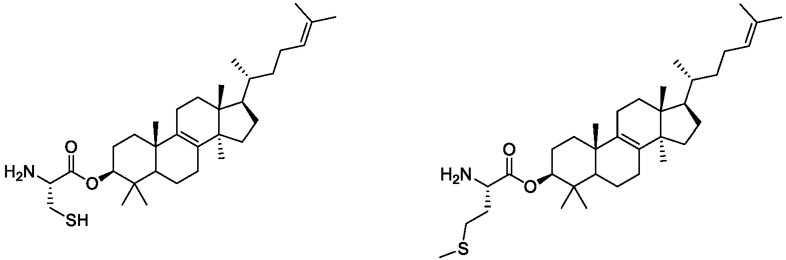 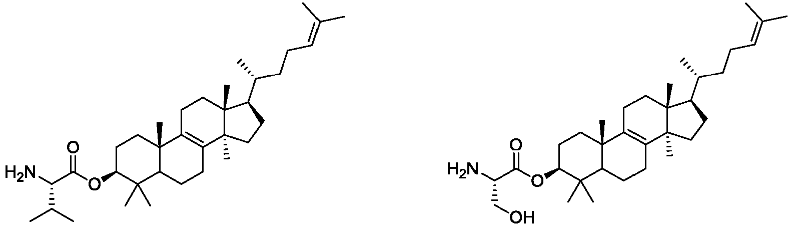 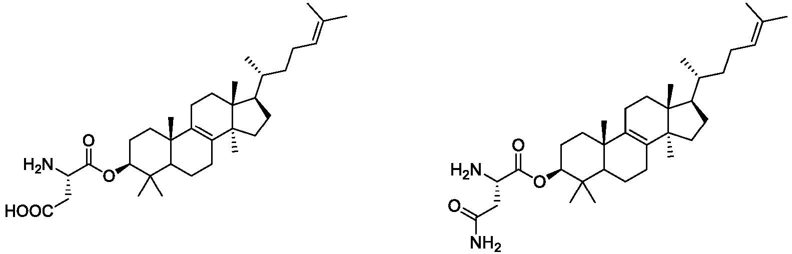 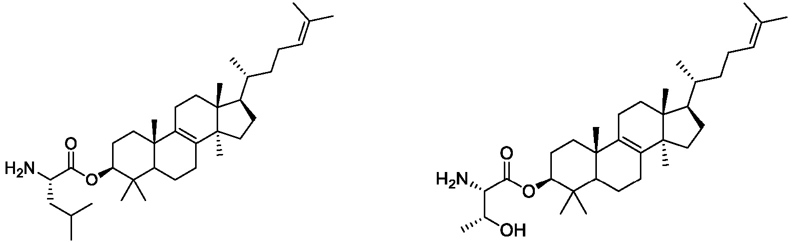 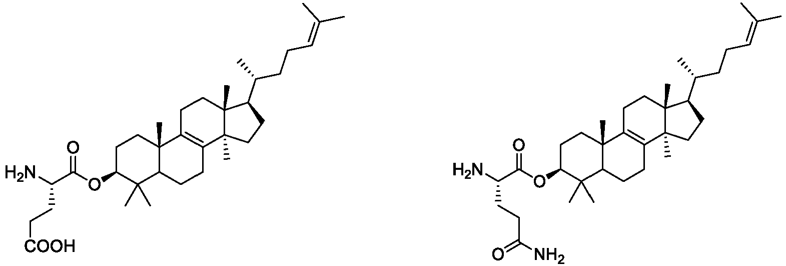 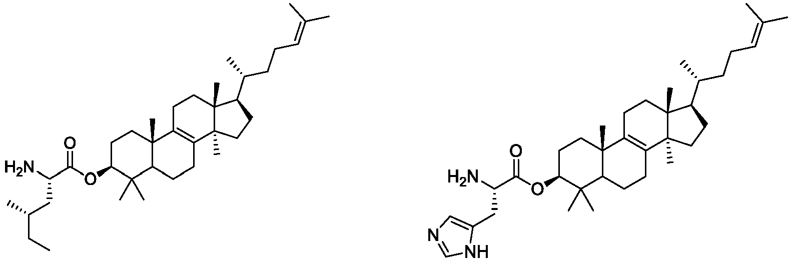 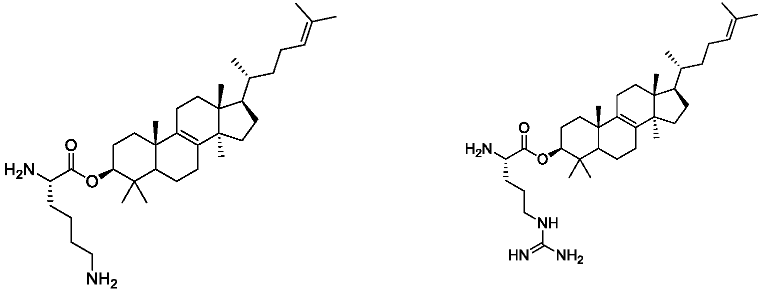 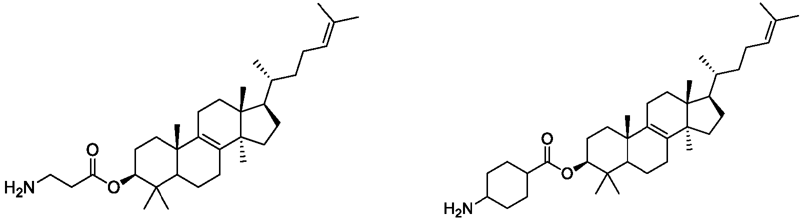 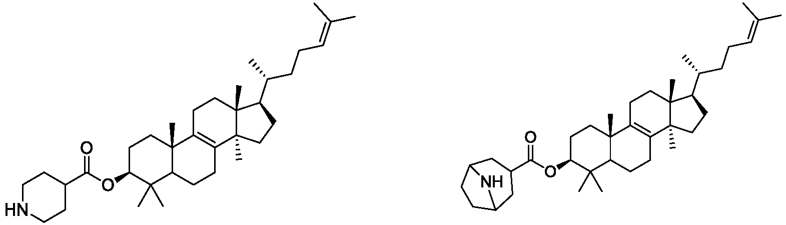 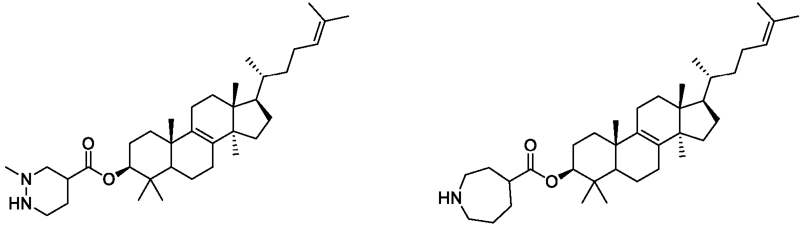 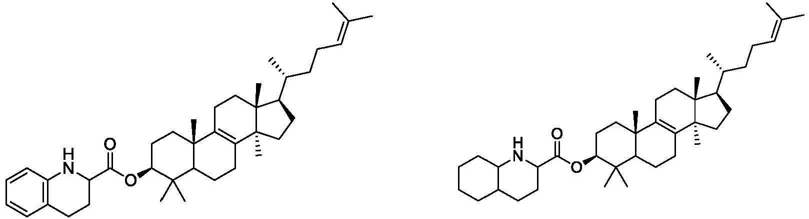 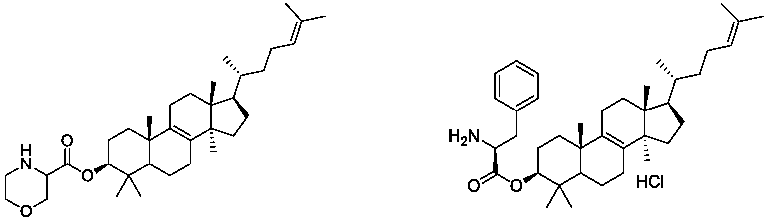 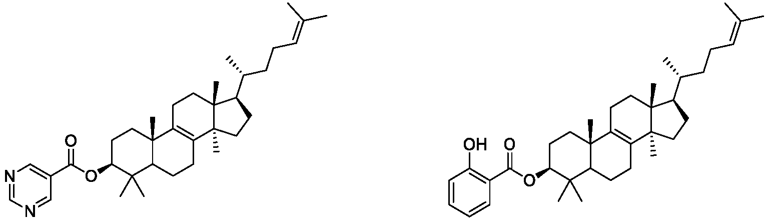 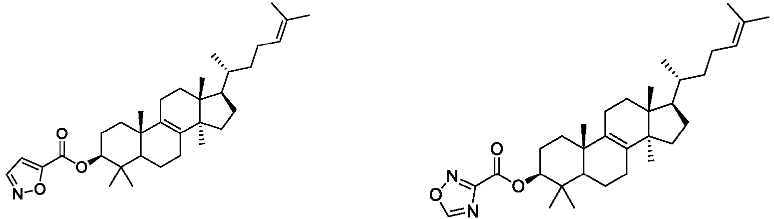 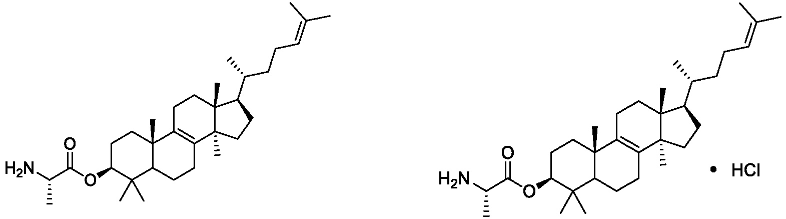 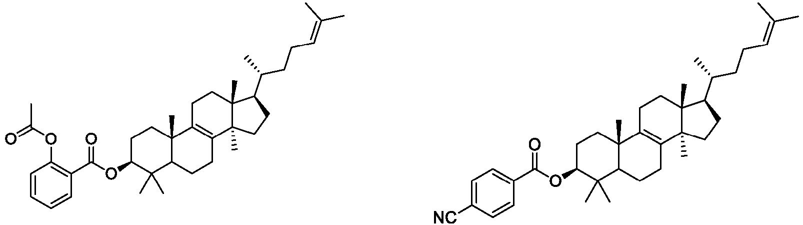 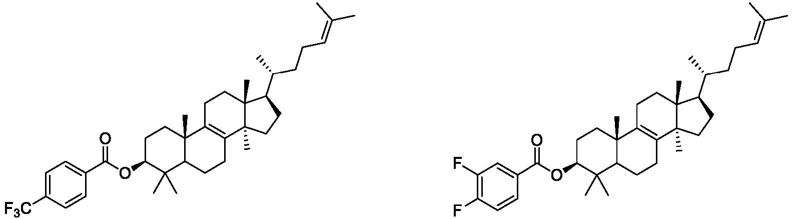 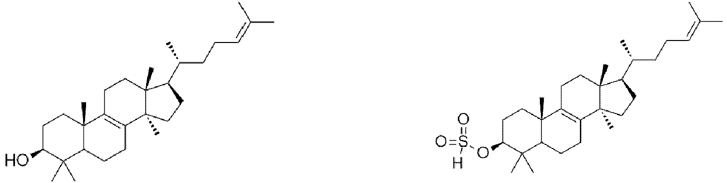 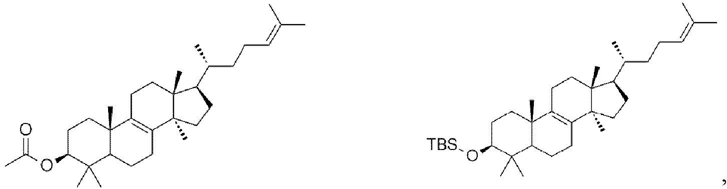 and

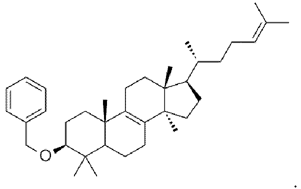

9. Use of a composition in the preparation of a medicament for preventing and/or treating presbyopia, wherein, the composition comprises the steroid compound according to any one of claims 1 to 8, and one or more of pharmaceutically acceptable carriers, excipients, diluents, adjuvants, or vehicles.

10. The use according to claim 9, wherein, the composition comprises: the steroid compound, hydroxypropyl methylcellulose, Poloxamer 407, Poloxamer 188, and water;
alternatively, the composition comprises: 10 to 40 parts by mass of the steroid compound, 110 to 120 parts by mass of hydroxypropyl methylcellulose, 2050 to 2060 parts by mass of Poloxamer 407, 160 to 170 parts by mass of Poloxamer 188, and 10,000 parts by mass of water.

11. A method for preventing and/or treating presbyopia, comprising administering of the steroid compound or the composition defined in the use according to any one of claims 1 to 10 to a subject in need thereof.
